**Europäisches Patentamt**

**European Patent Office**

**Office européen des brevets**

(19)

(11) Publication number: **0 586 255 A1**

## (12) EUROPEAN PATENT APPLICATION

(21) Application number: **93306964.3**

(22) Date of filing: **02.09.93**

(51) Int. Cl.⁵: **C12P 23/00, C12N 1/12,**
**// (C12P23/00, C12R1:89)**

The microorganism(s) has (have) been deposited with American Type Culture Collection under number(s) ATCC 75536.

(30) Priority: **03.09.92 JP 236176/92**

(43) Date of publication of application:
**09.03.94 Bulletin 94/10**

(84) Designated Contracting States:
**AT BE CH DE DK ES FR GB GR IE IT LI LU NL PT SE**

(71) Applicant: **TAKEDA CHEMICAL INDUSTRIES, LTD.**
**1-1, Doshomachi 4-chome**
**Chuo-ku, Osaka 541 (JP)**

(72) Inventor: **Matsumura, Shigeo**
**50-1, Yamadaminami**
**Suita, Osaka 565 (JP)**
Inventor: **Sumino, Yasuhiro**
**6-22, Higashiochiai 3-chome, Suma-ku**
**Kobe, Hyogo 654-01 (JP)**
Inventor: **Matsui, Junji**
**22-7, Shiginohigashi 2-chome, Joto-ku**
**Osaka 536 (JP)**

(74) Representative: **Laredo, Jack Joseph**
**Elkington & Fife Prospect House 8 Pembroke Road**
**Sevenoaks, Kent TN13 1XR (GB)**

(54) **Process for producing beta-carotene.**

(57) There is disclosed a process for producing β-carotene which comprises (a) cultivating a micro-alga which is tolerant to an active oxygen generator and capable of producing β-carotene to produce and accumulate β-carotene, and (b) collecting the β-carotene. The micro-alga is preferably Chlorella pyrenoidosa.

EP 0 586 255 A1

FIELD OF THE INVENTION

The present invention relates to an improved process for producing β-carotene. The process of the present invention can be used as an economical industrial process for producing natural β-carotene which has high safety.

β-Carotene has various useful activities such as vitamin effects as provitamin A, antioxidative activities (counteraction against active oxygen), immunopotentiation activities, preventive effects against cancer, preventive effects against harmful effects of ultraviolet, preventive effects against aging and the like [Functional Food Materials, Research and Development in Biologically Active Substances etc. Derived from Foods, p.156-175, Kogyogijutsu-kai (1989)).

BACKGROUND OF THE INVENTION

Processes for producing β-carotene and chlorophyll have been disclosed, for example, in the following literatures.

1) JP-A 3-183498 and 2) JP-A 57-83279:

The above literature 1) describes a process for producing β-carotene wherein freshwater algae belonging to the genera Chlamydomonas, Cylindrocystis, Stichococcus and Bracteacoccus are cultivated in a culture medium containing a halogenated alkaline metal (NaCl or KCl) in a concentration of at least 0.15M to accumulate β-carotene in the cells. The above literature 2) discloses that micro-algae increase the content of chlorophyll which is a photosynthetic pigment. However, these known literature references do not describe micro-algae tolerant to active oxygen.

Furthermore, microorganisms tolerant to active oxygen are described in, for example, the following literature.

3) JP-A 1-95776 and 4) JP-A 2-245187:

The above literature 3) describes a method of activating the peroxidase production efficiency of activated algae with active oxygen generators. The above literature 4) describes a process for producing superoxide dismutase using microorganisms. In the literature, Serratia marcescens and Escherichia coli B are described as the microorganisms. However, there is no description about Chlorella.

There is no description about micro-algae capable of producing β-carotene in either of the above literature references (3) and (4).

Since β-carotene is rich in dark green vegetables (e.g., spinach, broccoli, leaves of beefsteak plant, etc.) and yellow orange foods (e.g., carrot, pumpkin, citrus fruits, etc.), it can be supplied to some degree by ingesting such food. In Japan, the amount of ingestion of β-carotene is 1.5 mg to 2 mg per day. However, Food and Drug Administration and National Cancer Institute in U.S.A. have recommended ingestion of β-carotene in an amount of 6 mg per day, which is far more than the above amount (Clin. Nutr., vol. 7, p.118-122 (1988)).

This amount corresponds to 100 g of carrot which is known to have the high β-carotene content. As is clear from this, it is difficult to meet the above recommended amount with only dark green vegetables and yellow orange foods. Therefore, it is thought to supplement the ingestion of β-carotene with β-carotene itself or foods having high β-carotene contents to meet the above recommended amount.

Most of β-carotene marketed now are synthetic products. Natural β-carotene products occupy only a few percent. β-Carotene is used for foods, cosmetics, feed and the like. For example, it is added as a colorant to foods such as margarine, edible oils and fats, soft drinks and the like. For this purpose, cheap synthetic products are used. On the other hand, the natural products are used for enrichment of nutrition in view of the present nature-oriented trends. Therefore, the natural products are preferred for the above purpose when consideration is given to the safety of the synthetic products.

As described above, natural β-carotene is rich in vegetables and fruits . Further, it is contained in some microorganisms such as bacteria, yeast, mold, algae and the like. In comparison in terms of the percent contents, some species of these microorganisms greatly exceed vegetables and fruits. For example, since Dunaliella, a kind of green algae, accumulates β-carotene, β-carotene has been produced from Dunaliella. In addition, micro-algae such as Spirulina, Chlorella and the like are known to accumulate β-carotene In particular, Spirulina and Chlorella have been used as foods, particularly as health foods.

However, since Dunaliella and Spirulina cannot assimilate organic carbon sources, they are cultivated in the open air by photoautotrophic culture. The culture requires wide space, depends upon weather, and requires daylight. Therefore, the cell density dose not increase and the harvest is difficult. Further, contamination with natural enemies (e.g., Ciliophora, plankton, amoebae, etc.) or microorganisms cannot completely be prevented.

Therefore, an economical process for producing natural β-carotene in high yield and high purity is required.

## SUMMARY OF THE INVENTION

According to the present invention, there is provided:

1) process for producing β-carotene which comprises (a) cultivating a micro-alga which is tolerant to an active oxygen generator and capable of producing β-carotene to produce and accumulate β-carotene, and (b) collecting β-carotene; and

2) A pure culture of Chlorella pyrenoidosa tolerant to an active oxygen generator and capable of producing β-carotene.

## DETAILED DESCRIPTION OF THE INVENTION

The micro-algae to be used in the present invention which are tolerant to an active oxygen generator and capable of producing β-carotene have a single-cell generation, and they can be obtained by treating micro-algae capable of producing β-carotene with an active oxygen generator.

As examples of the micro-algae as the raw material which have a single-cell generation and are capable of producing β-carotene, there are algae belonging to the genera Chlorella (e.g., Chlorella pyrenoidosa, Chlorella vulgaris, Chlorella sorokiniana, Chlorella regularis, Chlorella salina, etc.), Chlamydomonas (e.g., Chlamydomonas reinhardtii, etc.), Dunaliella (e.g., Dunaliella salina, Dunaliella bardawil, Dunaliella tertiolecta, etc.), Euglena (e.g., Euglena gracilis, etc.), Ankistrodesmus (e.g., Ankistrodesmus braunie, etc.), Spirulina (e.g., Spirulina platensis, etc.), Microcystis (e.g., Microcystis aeruginosa, etc.), Synechococcus (e.g., Synechococcus sp., etc.) and the like. Among such micro-algae, algae belonging to the genus Chlorella are preferred. In particular, Chlorella pyrenoidosa and Chlorella vulgaris are preferably used. These micro-algae may be used as they are. The micro-algae preferably used are those subjected to mutagenesis treatments to contain β-carotene in high concentrations.

The mutagenesis treatments can be carried out by per se known methods such as by physical treatments or by the use of chemical substances. Examples of the methods of physical treatments are, methods using strong mutagens such as ultraviolet irradiation, gamma irradiation and the like. Examples of the methods using chemical substances are, methods using strong mutagens such as ethyl methanesulfonate, nitrosoguanidine, nitrous acid and the like; weak mutagens such as 2-aminopurine, 5-bromouracil, hydroxylamine and the like. In the present invention, safe and convenient mutagenesis treatments having strong mutability are preferably carried out. In particular, mutagenesis treatments by ultraviolet irradiation or with nitrosoguanidine are preferred.

Examples of the active oxygen generator are, generators of singlet oxygen, hydroxy radical, hydrogen peroxide, superoxide or the like. In particular, generators of singlet oxygen are preferred. Further, instead of using active oxygen generators, it is possible to use physical means such as microwave discharge to generate active oxygen. Examples of the active oxygen generator are, photosensitizers which generate singlet oxygen by visible light such as methylene blue, rose bengal, acridine orange, proflavine, thiopyronine, eosine yellow, lumiflavine, protoporphyrin and the like; photosensitizers which generate singlet oxygen by ultraviolet light such as NADH, α-terthienyl, furocoumarin, nalidixic acid, 6-mercaptopurine, tetracycline, bithionol and the like; hydrogen peroxide; sodium hypochlorite and the like. Among them, methylene blue, rose bengal and hydrogen peroxide are preferred.

The micro-algae which are tolerant to an active oxygen generator and capable of producing β-carotene can be prepared by per se known methods. Further, they can be prepared, for example, by the following method:

(1) Firstly, a micro-alga capable of producing β-carotene is cultivated. The medium may be a liquid medium or agar medium. The temperature for the cultivation is selected from temperatures suitable for the growth of the alga to be used, and is normally about 15°C to 45°C, preferably about 20°C to 40°C. The cultivation is continued for sufficient time for the growth of the alga to be used and the formation of β-carotene, and takes normally about 1 day to 14 days. The pH for the cultivation is adjusted to about 5 to 9, preferably about 6 to 8.

(2) Then, if necessary, mutagenesis treatments are carried out. Mutagenesis treatments by ultraviolet irradiation or with nitrosoguanidine are preferred, but other per se known methods can also be used.

(3) In order to increase the β-carotene content in micro-algae, a micro-alga subjected (or not subjected) to a mutagenesis treatment is cultivated if necessary. The medium is preferably a liquid medium, but may be an agar medium. The conditions for the cultivation are as described in (1) above. This step is not always necessary.

(4) The micro-alga is treated with an active oxygen generator. For example, the micro-alga is suspended in a buffer, an active oxygen generator in a given concentration is added, the resulting mixture is gently shaken, and sampling with the passage of time is carried out to observe the number of live cells. The general method for counting the number of the live cells is by spreading diluted solution of the sample on the agar medium, followed by counting the number of grown colonies. Alternatively, it is possible to count the number of live cells by determining photosynthesis activity. When light irradiation treatments are conducted, the cell concentration must be decreased for increasing the efficiency of the light irradiation.

Examples of the method wherein micro-algae are treated with an active oxygen generator other than the method of (4) above are, the method wherein an active oxygen generator is added to liquid media or agar media, the micro-algae are cultivated in the medium under conditions for the growth of the micro-algae, and the surviving micro-algae are selected. In this case, methods using agar media are most convenient. Liquid media are preferably used when the active oxygen generator is AAPH (2,2'-azobis(2-amidinopropane)-dihydrochloride), AMVN (2,2'-azobis(2,4-dimethylvaleronitrile)) or the like which generates active oxygen under heating conditions (about 37°C). Further, in the liquid culture including light irradiation treatments, attention must be paid to the initial cell concentration.

(5) The grown colonies are isolated from the part treated with an active oxygen generator where the number of the surviving colonies is decreased to not larger than about one tenth of the control (the number of the colonies of the part not treated with the active oxygen generator). By evaluating the β-carotene productivity, the micro-algae containing β-carotene in high concentration are selected.

The micro-algae can be used alone or in combination of two or more kinds.

The following embodiment illustrates the method to obtain a micro-alga tolerant to an active oxygen generator. Hereinafter, when micro-algae are used alone, they are sometimes referred to as algal cells.

Chlorella pyrenoidosa UTEX 1663 (obtained from the collection of University of Texas, Austin, U.S.A. (UTEX COLLECTION)) belonging to the genus Chlorella capable of producing β-carotene was cultivated at 35°C for 3 days in the agar medium (A) described in Example 1 hereinafter. The resulting algal cells were suspended in a buffer of potassium phosphate (M/15, pH 7.0). Then nitrosoguanidine (NTG) was added to a final concentration of 50 μg/ml. The mixture was gently shaken at 28°C for 15 minutes for mutagenesis treatment. Then the supernatant was removed by centrifugation. Again, the above buffer was added, and the resulting mixture was shaken and subjected to centrifugation to wash the algal cells. The surviving ratio of the algal cells at this time was about 5%. Methylene blue (1 mg/ml), an active oxygen generator, was added to the above agar medium (A) in Example 1. The above NTG-treated algal cells were spread on the resulting agar plate, and cultivated at 35°C for 7 days. By evaluating the β-carotene productivity of the resulting colonies, algal cells containing β-carotene in high concentrations were selected. The algal cells thus obtained are referred to as Chlorella pyrenoidosa MB 115.

As described above, the micro-algae containing β-carotene in high concentrations can be obtained through the following steps: (i) the mutagenesis treatments, (ii) the acquisition of the micro-algae tolerant to an active oxygen generator, and (iii) the evaluation of the β-carotene productivity by using the acquired micro-algae. Further, micro-algae containing β-carotene in higher concentrations can be obtained by repeating the above (i) to (ii) steps while increasing the concentration of the active oxygen generator.

The following is the appearances of the culture and physiological characteristics of Chlorella pyrenoidosa MB 115 described above.

1) Appearances of the culture:

Chlorella pyrenoidosa MB 115 formed green colonies of 2 to 3 mm diameter on the agar medium (A) in Example 1 hereinafter by the culture at 35°C for 5 days.

The colonies formed by liquid culture at 35°C were observed with a microscope. They were green single cells of 2.5 to 5 mm diameter in the rice-shape to global shape. These characteristics were substantially the same as those of the parent strain, Chlorella pyrenoidosa UTEX 1663.

2) Physiological characteristics:

When cultivated at 20 to 50°C for 3 days in the agar medium (A) in Example 1, Chlorella pyrenoidosa MB 115 was found to grow at 20 to 41°C.

As the carbon source, glucose, fructose, galactose and acetic acid are used. In particular, glucose is preferably used. As the nitrogen source, ammonium sulfate, urea, sodium nitrate are used.

These characteristics were substantially the same as those of the parent strain, <u>Chlorella</u> <u>pyrenoidosa</u> UTEX 1663.

The culture of the micro-algae tolerant to an active oxygen generator and capable of producing β-carotene can be carried out by chemoheterotrophic culture using glucose as the carbon source, photoautotrophic culture, mixotrophic culture by the combined use of them. Thus, the micro-algae containing β-carotene high concentrations can be obtained. The chemoheterotrophic culture and mixotrophic culture are preferred in the culture under a closed system such as tank culture.

The culture can be carried out continuously or intermittently by conventional static culture, shake culture, aeration and agitation culture or solid culture. Any medium can be used as long as the microorganisms to be used can grow in the medium. The composition of the medium may be that conventionally used.

The carbon source for the chemoheterotrophic culture or mixotrophic culture can appropriately be selected from carbohydrates (e.g., glucose, lactose, sucrose, maltose, etc.), fats and oils (e.g., soybean oil, lard oil, chicken oil, etc.), fatty acids (e.g., palmitic acid, stearic acid, oleic acid, etc.), organic acids (e.g., acetic acid, propionic acid, etc.) and alcohols (e.g., ethyl alcohol, glycerin, etc.) each of which is assimilable. These carbon sources can be used alone or in combination thereof. On the other hand, sodium bicarbonate or aeration of carbon dioxide gas is used in the photoautotrophic culture. As the nitrogen source, there can be used organic nitrogen sources such as peptone, soybean flour, cotton seed flour, corn steep liquor, yeast extract, meat extract, malt extract, whey, urea, guanidine hydrochloride or the like; as well as inorganic nitrogen sources such as ammonium sulfate, ammonium chloride, ammonium nitrate, ammonium phosphate, potassium nitrate or the like. These nitrogen sources may be used alone or, if necessary, in combination thereof. To the medium, in addition to the above carbon sources and nitrogen sources, there may be added factors necessary for the growth such as minerals (e.g., calcium, iron, magnesium, etc.), amino acids (e.g., glutamic acid, aspartic acid, alanine, lysine, methionine, etc.), vitamins (e.g., $B_1$, $B_2$, nicotinic acid, $B_{12}$, C, etc.) or the like; growth promoting substances; metal-chelating agents (e.g., hemin, chlorophyll, etc.) or the like. It is effective to add caustic alkalis, a solution of sodium carbonate, calcium salts or the like, or antifoaming agents in order to control the pH and foam during the culture. Particularly preferred results are sometimes obtained by adding an inhibitor of carotenoid biosynthesis (e.g., norflurazon).

The temperature for the cultivation can be selected from temperatures suitable for the growth of the algae to be used, and is normally about 15°C to 45°C, preferably about 20°C to 40°C. The cultivation is continued for sufficient time for the growth of the algae to be used and the formation of β-carotene. It takes normally 1 day to 14 days. The pH for the cultivation is adjusted to about 5 to 9, preferably about 6 to 8.

β-Carotene may be collected from the micro-algae by methods conventionally used in the art. For example, β-carotene is extracted by using oils and fats or organic solvents or the like from the micro-algae as they are, or from those after treatments such as freeze-thawing, trituration, sonication, treatments by osmotic pressure, lysis of the cell walls, treatments with surfactants or the like. Alternatively, it can be extracted by means such as supercritical extraction. Further, instead of collecting β-carotene from the micro-algae, the micro-algae as such may be used as the micro-algae having high β-carotene content for foods (e.g., noodles, breads, confectioneries, juices, etc.) or feed (e.g., feed for fish cultivation, feed for sericulture, feed for poultries, etc.) after sterilization such as thermal sterilization, sterilization with chemicals or the like.

As described hereinabove, according to the present invention, natural β-carotene having high safety can be produced in high yield and high purity.

The following examples further illustrate the present invention in detail but are not to be construed to limit the scope thereof.

## Example 1

<u>Chlorella</u> <u>pyrenoidosa</u> UTEX 1663 (obtained from the collection of University of Texas, Austin, U.S.A. (UTEX COLLECTION)) was spread on the agar medium (A) shown in Table 2 below, and grown at 35°C for 3 days. Then, the algal cells thus obtained were suspended in potassium phosphate buffer (M/15, pH 7.0). Nitrosoguanidine (NTG) was added to a final concentration of 50 μg/ml. The mixture was gently shaken at 28°C for 15 minutes. Then, the supernatant was removed by centrifugation. Again, the above buffer was added, and the mixture was shaken and centrifuged to wash the algal cells. The survival ratio of the algal cells at that time was about 5 %. Methylene blue (1 mg/l) was added to the agar medium (A). The algal cells obtained above were spread on the resulting agar plate, and cultivated at 35°C for 7 days. <u>Chlorella</u> <u>pyrenoidosa</u> MB 115 was selected from the resulting colonies. The tolerance of <u>Chlorella</u> <u>pyrenoidosa</u> MB 115 and its parent strain, <u>Chlorella</u> <u>pyrenoidosa</u> UTEX 1663, to methylene blue was determined by the method for measuring tolerance based on relative growth shown below. The results are shown in Table 1 below.

Table 1

| Tolerance to methylene blue (%) | | | |
|---|---|---|---|
| Chlorella pyrenoidosa | Methylene blue concentration ($\mu$g/ml) | | |
| | 0 | 1.56 | 3.13 |
| UTEX | 100 | 0.75 | 0.25 |
| MB 115 | 100 | 91.1 | 107 |

The method for measuring tolerance based on relative growth:

The tolerance (%) of the micro-algae to an active oxygen generator can be determined follows. A given amount of the active oxygen generator described in Examples or Experiments was added to the medium (B) shown in Table 2 to obtain medium (C). Algal strains for the test grown on the agar medium (A) shown in Table 2 were inoculated in test tubes containing the medium (B) or (C) (each 5 ml) so that the absorbance at 620 nm became 0.01. They are cultivated with shaking at 35°C for 5 days. The culture thus obtained was diluted 100-fold with water. Growth was determined by measuring absorbance at 620 nm ($B_{620}$ and $C_{620}$). The relative growth of the test algal strains in the medium (C) to which an active oxygen generator was added was calculated from the following equation.

$$\text{Relative growth (\%)} = (C_{620}/B_{620}) \times 100$$

This relative growth was defined as the tolerance (%).

Table 2

| Composition of the medium | | |
|---|---|---|
| Ingredients | Concentration (g/liter) | |
| | (Concentration in the raw solution) | |
| | Agar medium (A) | Liquid medium (B) |
| Glucose (Separate sterilization: 123°C, 20 min.) | 10.0 | 20.0 |
| Potassium dihydrogenphosphate | 2.5 | 2.5 |
| Dipotassium hydrogenphosphate | 2.5 | 2.5 |
| Trisodium citrate | 1.0 | 1.0 |
| Magnesium sulfate heptahydrate | 0.8 | 0.8 |
| Urea | 0.8 | 0.8 |
| Trace metal slats etc. (See Table 3) | See Table 3 | See Table 3 |
| Yeast extract | 2.0 | - |
| Powdery agar | 16 | - |

Table 3

| Ingredients of the trace metal salts etc. | |
|---|---|
| Ingredients | Concentration (mg/liter) (Concentration in the raw solution) |
| Boric acid | 114.2 |
| Calcium chloride | 83.5 |
| Ferric sulfate heptahydrate | 49.8 |
| Zinc (II) sulfate heptahydrate | 88.2 |
| Manganese (III) sulfate tetrahydrate | 14.4 |
| Sodium molybdate | 7.1 |
| Cupric sulfate pentahydrate | 15.7 |
| Cobalt (III) chloride hexahydrate | 4.9 |
| conc. Hydrochloric acid | 0.2 (ml/liter) |

Example 2

Chlorella pyrenoidosa MB 115 was inoculated in the agar medium (A) shown in Example 1, and cultivated at 35°C for 3 days. Then, one loopful of this algal cells was inoculated in a 200 ml Erlenmeyer flask containing a medium (30 ml) for the seed culture having the same composition as that of the liquid medium (B), and cultivated at 35°C for 2 days with shaking. This culture medium (1.5 ml) was transferred to a 200 ml Erlenmeyer flask containing a medium (30 ml) for the main culture having the same composition as that of the liquid medium (B). After cultivation for 4 days, the amount of the algal cells and β-carotene in the culture medium was determined.

The amount of the algal cells was determined as follows. The algal cells were collected from the culture medium (1 ml) by centrifugation. Distilled water (1 ml) was added, and the mixture was agitated and centrifuged again for washing. After lyophilization, the weight of the algal cells was measured.

The amount of β-carotene was determined as follows. The algal cells were collected from the culture medium (1 ml) by centrifugation (1,800 rpm, 20 minutes). To the algal cells thus obtained, an aqueous solution (10N, 1 ml) of potassium hydroxide containing 2.5 (W/W) % ascorbic acid was added. The resulting mixture was stirred, and then allowed to stand at 60°C for 10 minutes. Next, methanol (19 ml) was added and mixed well. The mixture thus obtained (4 ml) was transferred to a test tube with stopper. n-Hexane (2 ml) was added to the test tube with stopper. The mixture was stirred and allowed to stand for separating layers. The upper layer was removed. An aqueous 20 (w/w) % solution (8 ml) of sodium chloride was added to the residue. The resulting mixture was stirred, and then extracted three times with ethyl ether (4 ml). Anhydrous sodium sulfate was added to the extracted ethyl ether solution for dehydration. Then sampling was done (4 ml). The solution was concentrated to dryness under reduced pressure. The residue was dissolved in n-hexane again. The solution was used as a sample. The sample thus obtained was filtered (DISMIC-3JP, manufactured by Advantec, Japan), and then the amount of β-carotene was determined by high performance liquid chromatography (HPLC). For the HPLC, Novapack $C_{18}$ (8 X 100 mm) (manufactured by Nihon Millipore Limited, Japan) column was used. The mobile phase used was methanol/ethyl acetate (the mixing ratio: 8/2). The column temperature was 40°C and the flow rate was 2 ml/minute. As a result, the β-carotene content in Chlorella pyrenoidosa MB 115 was 1.0 mg per mg of the algal cells.

On the other hand, the β-carotene content in Chlorella pyrenoidosa UTEX 1663 was determined by the same method described above. As a result, the β-carotene content in Chlorella pyrenoidosa UTEX 1663 was 0.48 mg per 1 g of the algal cells.

## Example 3

Chlorella pyrenoidosa MB 115 was used as seed algae. One loopful of the algae was inoculated in a 200 ml Erlenmeyer flask containing a medium (30 ml) for the seed culture having the same composition as that of the liquid medium (B) shown in Example 1, and cultivated at 35°C for 2 days with shaking (primary seed culture). This culture medium (6.5 ml) was transferred to a 1000 ml Erlenmeyer flask containing a medium (130 ml) for the seed culture, and cultivated at 35°C for 2 days (second seed culture).

The culture medium thus obtained (125 ml) was transferred to a 5 liter jar fermentor containing a medium (2.5 liter) for the main culture having the same composition as that of the liquid medium (B), and cultivated for 90 hours. As a result, 30 mg/liter of β-carotene was accumulated. The amount of the algal cells at this time was 30 g/liter.

## Experiment 1

The tolerance (%) of Chlorella pyrenoidosa MB 115

Chlorella pyrenoidosa MB 115 was examined for the sensitivity to several active oxygen generators. The following results were obtained. That is, Chlorella pyrenoidosa MB 115 , which was a strain capable of highly producing β-carotene produced by the methylene blue tolerance, exhibited tolerance not only to rose bengal, a generator of singlet oxygen, but also to duroquinone, a generator of a superoxide anion, and hydrogen peroxide. This shows that the use of any of these active oxygen generators gives strains tolerant to different active oxygen generators.

The results of the experiment were as follows.

### 1) Duroquinone

The tolerances of Chlorella pyrenoidosa UTEX 1663 (obtained from the collection of University of Texas, Austin, U.S.A. (UTEX COLLECTION)) and Chlorella pyrenoidosa MB 115 was determined by the method for measuring tolerance based on relative growth. The results are shown in Table 4.

Table 4

| Tolerance (%) to duroquinone | | |
|---|---|---|
| Chlorella pyrenoidosa | Duroquinone concentration ($\mu$M) | |
| | 0 | 5 |
| UTEX 1663 | 100 | 0 |
| MB 115 | 100 | 113 |

### 2) Hydrogen peroxide

Hydrogen peroxide was added to a final concentration of 20 $\mu$g/ml to potassium phosphate buffer (M/15, pH 7,0) containing about 1.0 x 106 C.F.U./ml of cells of Chlorella pyrenoidosa UTEX 1663 (obtained from the collection of University of Texas, Austin, U.S.A. (UTEX COLLECTION)) or Chlorella pyrenoidosa MB 115. After treatment at 25°C for 2 hours, the mixture was diluted and spread on the agar medium (A) in Example 1. Then, the tolerance was determined. The results shown in Table 5 were obtained. The survival ratio at 0 hour after the treatment (no hydrogen peroxide was added) was reckoned to be 100%. The tolerances are indicated as relative survival ratios to the above survival ratio.

Table 5

| Tolerance (%) to hydrogen peroxide | | |
|---|---|---|
| Chlorella pyrenoidosa | Hydrogen peroxide concentration (µg/ml) | |
| | 0 | 20 |
| UTEX 1663 | 100 | 0.1 |
| MB 115 | 100 | 1.3 |

3) Rose bengal

Rose bengal was added to a final concentration of 5 µM to potassium phosphate buffer (M/15, pH 7.0) containing about $1.0 \times 10^6$ C.F.U./ml of cells of Chlorella pyrenoidosa UTEX 1663 (obtained from the collection of University of Texas, Austin, U.S.A. (UTEX COLLECTION)) or Chlorella pyrenoidosa MB 115. After light irradiation (ca. 10,000 lux) at 25°C for 1.5 hours, the mixture was diluted and spread on the agar medium (A) in Example 1. Then, the tolerance was determined. The results shown in Table 6 were obtained. The survival ratio at 0 hour after the treatment (no rose bengal was added) was reckoned to be 100%. The tolerances are indicated as relative survival ratios to the above survival ratio.

Table 6

| Tolerance (%) to rose bengal | | |
|---|---|---|
| Chlorella pyrenoidosa | Rose bengal concentration (µM) | |
| | 0 | 5 (light irradiation) |
| UTEX 1663 | 100 | 0.36 |
| MB 115 | 100 | 0.85 |

Example 4

The suspension of Chlorella pyrenoidosa MB 115 (about $1.0 \times 10^8$ C.F.U./ml) was treated with Nitrosoguanidine (NTG) (the time of the treatment was 10 minutes) and washed by the method described in Example 1. The algal cells (1 ml) thus obtained were transferred to a 200 ml Erlenmeyer flask containing a 30 ml of the liquid medium (B) shown in Table 2 and cultivated at 35°C for 2 days with shaking. The culture medium thus obtained was added to a rose bengal solution (rose bengal 5 µM, phosphate buffer M/15, pH 7.0) so that the absorbance at 620 nm became 0.6, and gently shaken at 25°C for 1.5 hours with light irradiation (ca. 13,000 lux). The suspension treated with rose bengal was centrifuged. The algal cells thus obtained were wased one time with almost same volume of phosphate buffer (M/15, pH 7.0) and suspended in the phosphate buffer described above. This suspension was diluted, spread on the agar medium (A) shown in Table 2, and cultivated at 35C° for 5 days. 100 colonies were separated from the grown colonies and the β-carotene productivity thereof was evaluated. As a result, Chlorella pyrenoidosa RB 35, which was superior to its parent strain, Chlorella pyrenoidosa MB 115, in β-carotene productivity, was obtained. The β-carotene contents of them were shown in Table 7.

On the other hand, cultivation was carried out as follows. Cultivation (seed culture) was carried out in a 200 ml Erlenmeyer flask containing the liquid medium (B) (30 ml) for 2 days. The culture medium thus obtained (2 ml) was transferred to a 200 ml Erlenmeyer flask containing a liquid medium (D) (200 ml) having the same composition as that of the liquid medium (B) except that the concentrations (g/liter) of glucose, urea and magnesium sulfate heptahydrate were increased to 80, 7.0 and 2.0, respectively, and was cultivated with shaking for 6 days.

table 7

| β-Carotene contents | | |
|---|---|---|
| Chlorella pyrenoidosa | β-Carotene contents | |
| | (mg/liter culture medium) | (mg/g algal cells) |
| MB 115 | 28.5 | 0.92 |
| RB 35 | 33.6 | 1.1 |

Example 5

Regarding β-carotene production, strain improvements of Chlorella pyrenoidosa MB 115 were carried out by repeating 7 times the method described in Example 4. Chlorella pyrenoidosa RB 2-13 thus thus obtained was treated as follows.

After the process of NTG treatment and rose bengal treatment described in Example 4, 400 colonies from grown colonies were evaluated in β-carotene productivity. More details are described below. The process comprising mutagenesis treatment, liquid cultivation, rose bengal treatment and liquid cultivation was repeated twice . In the third time, the algal cells were washed with phosphate buffer once, diluted, spread on the agar medium (A), and then cultivated. 400 colonies from grown colonies were separated and β-carotene productivity thereof was evaluated. As a result, Chlorella pyrenoidosa RB 3-356 (ATCC No. 75536 ), which was superior to its parent strain, Chlorella pyrenoidosa RB 2-13, in β-carotene productivity, was obtained. The β-carotene contents of both Chlorella pyrenoidosa RB 2-13 and Chlorella pyrenoidosa RB 3-356 (ATCC No. 75536 ) were shown in Table 8.

The appearances of the culture and physiological characteristics of Chlorella pyrenoidosa RB 3-356 (ATCC No. 75536 ) were substantially the same as those of Chlorella pyrenoidosa UTEX 1663. The growth rate of Chlorella pyrenoidosa RB 3-356 (ATCC No. 75536 ) was slightly lower than Chlorella pyrenoidosa UTEX 1663, and cells of Chlorella pyrenoidosa RB 3-356 (ATCC No. 75536 ) were yellowish green while those of Chlorella pyrenoidosa UTEX 1663 were dark green.

Table 8

| β-Carotene contents | | |
|---|---|---|
| Chlorella pyrenoidosa | β-Carotene contents | |
| | (mg/liter culture medium) | (mg/g algal cells) |
| RB 3-356 | 132 | 4.4 |
| RB-2-13 | 89.2 | 2.7 |

Claims

1. A process for producing β-carotene which comprises (a) cultivating a micro-alga which is tolerant to an active oxygen generator and capable of producing β-carotene to produce and accumulate β-carotene, and (b) collecting the β-carotene.

2. A process according to claim 1, wherein the active oxygen generator is a generator of singlet oxygen.

3. A process according to claim 1, wherein the active oxygen generator is methylene blue.

4. A process according to claim 1, wherein the active oxygen generator is rose bengal.

5. A process according to claim 1, wherein the micro-alga is an alga belonging to the genus Chlorella.

6. A process according to claim 5, wherein the micro-alga is <u>Chlorella</u> <u>pyrenoidosa</u>.

7. A process according to claim 6, wherein the micro-alga is <u>Chlorella</u> <u>pyrenoidosa</u> MB 115.

8. A pure culture of <u>Chlorella</u> <u>pyrenoidosa</u> tolerant to an active oxygen generator and capable of producing β-carotene.

9. pure culture according to claim 8 which is <u>Chlorella</u> <u>pyrenoidosa</u> MB 115.

10. A pure culture according to claim 8 which is <u>pyrenoidosa</u> RB 3-356 (ATCC No. 75536 ).

**European Patent Office**

## EUROPEAN SEARCH REPORT

Application Number

EP 93 30 6964

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 052 777  F. HOFFMANN-LA ROCHE & CO. Claims --- | 1 | C 12 P   23/00 C 12 N    1/12   // (C 12 P   23/00 C 12 R    1:89 ) |
| D,X | PATENT ABSTRACTS OF JAPAN, unexamined applications, C field, vol. 15, no. 436, November 7, 1991 THE PATENT OFFICE JAPANESE GOVERNMENT page 23 C 882 No. 03-183 498 (NIPPON ZEON CO LTD) --- | 1 | |
| D,A | PATENT ABSTRACTS OF JAPAN, unexamined applications, C field, vol. 6, no. 169, September 2, 1982 THE PATENT OFFICE JAPANESE GOVERNMENT page 21 C 122 No. 57-83 279 (BRIDGESTONE TIRE) ----- | 8 | |

TECHNICAL FIELDS SEARCHED (Int. Cl.5)

C 12 P
C 12 N

The present search report has been drawn up for all claims

| Place of search | Date of completion of the search | Examiner |
|---|---|---|
| THE HAGUE | 09-11-1993 | SCHWICKERATH F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)